(19)

Europäisches
Patentamt

European
Patent Office

Office européen
des brevets

(11) **EP 4 394 782 A1**

(12) **EUROPEAN PATENT APPLICATION**

(43) Date of publication:
**03.07.2024 Bulletin 2024/27**

(21) Application number: **23204948.6**

(22) Date of filing: **20.10.2023**

(51) International Patent Classification (IPC):
**G16C 60/00** (2019.01)

(52) Cooperative Patent Classification (CPC):
**G16C 60/00**; G16C 10/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **29.12.2022 IN 202221076873**

(71) Applicant: **Tata Consultancy Services Limited
Maharashtra (IN)**

(72) Inventors:
• **KEDIA, PRIYA**
  **411057 Maharashtra (IN)**
• **GUPTA, RAKESH**
  **411013 Maharashtra (IN)**
• **RAI, BEENA**
  **411013 Maharashtra (IN)**
• **KAUSLEY, SHANKAR BALAJIRAO**
  **411013 Maharashtra (IN)**
• **BADHE, YOGESH KAILAS**
  **411013 Maharashtra (IN)**

(74) Representative: **Goddar, Heinz J.
Boehmert & Boehmert
Anwaltspartnerschaft mbB
Pettenkoferstrasse 22
80336 München (DE)**

(54) **METHOD AND SYSTEM FOR DESIGN OF EDIBLE POLYMER FILMS FOR COATING OF PERISHABLE FOOD ITEMS**

(57) State of the art mechanisms for edible food coating design have the disadvantage that they fail to accommodate various factors such as type of compounds, chemical composition, and processing parameters such as pH, drying rate, temperature affect the nano-microstructure of the film. The disclosure herein generally relates to edible food coating, and, more particularly, to a method and system for developing and characterizing edible films using molecular dynamic simulations. The molecular dynamic simulations help design the edible food coatings, which may be further used for practical applications.

FIG. 2

EP 4 394 782 A1

**Description**

CROSS-REFERENCE TO RELATED APPLICATIONS AND PRIORITY

The present application claims priority to Indian application no. 202221076873, filed on December 29, 2022.

TECHNICAL FIELD

The disclosure herein generally relates to edible food coating, and, more particularly, to a method and system for developing and characterizing edible films using molecular dynamic simulations.

BACKGROUND

Perishable food items are coated with edible films as they provide an inexpensive, biodegradable way of packaging which also extends the shelf life of perishable food items. These films are generally made up of various kind of polymers and applied on various class of food items. The effectiveness of the film is not controlled by its material properties. It is a complex situation where materials compatibility (food item and film), environment and operating condition also play an important role. Due to this, researchers have developed and reported several kinds of edible films using different kind of chemical compounds, and their relative composition.

In state of the art mechanisms, detailed experimentation is performed to first develop each sample that can possibly be made by varying the chemical compounds and their compositions. Later, a detailed experimental characterization of film is performed by measuring properties such as gas permeability, mechanical strength, anti-microbial activity, and antioxidant properties to name a few. Finally, the testing of the films is carried out at several external environmental conditions. The overall goal is to make an edible coating, strong enough to hold on food items and porous enough to allow transport of oxygen and harmful gases across it. The transport of these gases, one of the critical phenomena determining the effectiveness of the films, is quantified in terms of diffusivity and permeability which are highly dependent on the nano/microstructure of the film. Various factors such as type of compounds (polymers, solvent, etc.), chemical composition, and processing parameters such as pH, drying rate, temperature affect the nano-microstructure of the film.

SUMMARY

Embodiments of the present disclosure present technological improvements as solutions to one or more of the above-mentioned technical problems recognized by the inventors in conventional systems. For example, in one embodiment, a processor implemented method is provided. In this method, a user chosen intent is received via one or more hardware processors, wherein the user chosen intent comprises one of (i) designing of the edible film (ii) characterization of the edible film. Further, one or more specifications are received, via the one or more hardware processors, from the user, based on the user chosen intent, wherein the one or more specifications comprise (i) a plurality of design specifications associated with the designing of the edible film, and (ii) a plurality of characterization specifications associated with the characterization of the edible film. Further, a plurality of tunable design factors are assigned via the one or more hardware processors, from the one or more design specifications with associated range of values, based on the user chosen intent. Further, a Design of Experiments (DOE) is generated via the one or more hardware processors, by using a plurality of combinations of the plurality of tunable design factors, wherein the DOE generates a plurality of edible film designs. Further, a molecular model representing a dried version of the edible film is constructed via the one or more hardware processors, for one or more of the plurality of edible film designs. Further, values of one or more properties of the molecular model representing dried film for the one or more of the plurality of edible film designs is estimated using molecular simulations, via the one or more hardware processors, where a list of film properties is provided by user in specifications. Further, if the chosen user intent is designing of film, an optimum value for each of the plurality of tunable design factors, satisfying one or more value requirements of one or more properties of the edible film provided by the user during specifications, is identified via the one or more hardware processors.

In another aspect, a system is provided. The system includes one or more hardware processors, a communication interface, and a memory storing a plurality of instructions. The plurality of instructions when executed, cause receiving a user chosen intent, wherein the user chosen intent comprises one of (i) designing of the edible film (ii) characterization of the edible film. Further, one or more specifications are received, via the one or more hardware processors, from the user, based on the user chosen intent, wherein the one or more specifications comprise (i) a plurality of design specifications associated with the designing of the edible film, and (ii) a plurality of characterization specifications associated with the characterization of the edible film. Further, a plurality of tunable design factors are assigned via the one or more hardware processors, from the one or more design specifications with associated range of values, based on the user chosen intent. Further, a Design of Experiments (DOE) is generated via the one or more hardware processors, by using a plurality of combinations of the plurality of tunable design factors, wherein the DOE generates a plurality of edible film designs. Further, a molecular model representing a dried version of the edible film is constructed via the one or more hardware processors, for one or more of the plurality of edible film designs. Further, values of one or more properties of the molecular model representing dried film for the one or more of the plurality of edible film designs is estimated using molecular simulations, via the one or more hardware processors, where a list of film properties is provided by user in specifications. Further, if the chosen user intent is designing of film, an optimum value for each of the plurality of tunable design factors, satisfying one or more value requirements of one or more properties of the edible film provided by the user during specifications, is identified via the one or more hardware processors.

In yet another aspect, a non-transitory computer readable medium is provided. The non-transitory computer readable medium includes a plurality of instructions, which when executed, cause one or more hardware processors to perform the following steps. Initially, a user chosen intent is received via one or more hardware processors, wherein the user chosen intent comprises one of (i) designing of the edible film (ii) characterization of the edible film. Further, one or more specifications are received, via the one or more hardware processors, from the user, based on the user chosen intent, wherein the one or more specifications comprise (i) a plurality of design specifications associated with the designing of the edible film, and (ii) a plurality of characterization specifications associated with the characterization of the edible film. Further, a plurality of tunable design factors are assigned via the one or more hardware processors, from the one or more design specifications with associated range of values, based on the user chosen intent. Further, a Design of Experiments (DOE) is generated via the one or more hardware processors, by using a plurality of combinations of the plurality of tunable design factors, wherein the DOE generates a plurality of edible film designs. Further, a molecular model representing a dried version of the edible film is constructed via the one or more hardware processors, for one or more of the plurality of edible film designs. Further, values of one or more properties of the molecular model representing dried film for the one or more of the plurality of edible film designs is estimated using molecular simulations, via the one or more hardware processors, where a list of film properties is provided by user in specifications. Further, if the chosen user intent is designing of film, an optimum value for each of the plurality of tunable design factors, satisfying one or more value requirements of one or more properties of the edible film provided by the user during specifications, is identified via the one or more hardware processors.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary system for design of edible polymer films for coating of perishable food items, according to some embodiments of the present disclosure.
FIG. 2 is a flow diagram depicting steps involved in the process of design and characterization of edible polymer films for coating of perishable food items, using the system of FIG. 1, according to some embodiments of the present disclosure.
FIG. 3 is a flow diagram depicting steps involved in the process of performing a drying process associated with the design of edible polymer films for coating of perishable food items, using the system of FIG. 1, according to some embodiments of the present disclosure.
FIG. 4 is a flow diagram depicting steps involved in the process of identifying an optimum value for each of the plurality of tunable design factors, associated with the design of edible polymer films for coating of perishable food items, using the system of FIG. 1, according to some embodiments of the present disclosure.
FIGS. 5 through 7C are example diagrams showing experimental results of the process of design and characterization of edible polymer films for coating of perishable food items, using the system of FIG. 1, according to some embodiments of the present disclosure.

DETAILED DESCRIPTION OF EMBODIMENTS

Exemplary embodiments are described with reference to the accompanying drawings. In the figures, the leftmost digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

In state of the art mechanisms for designing edible food coatings, detailed experimentation is performed to first develop each of the sample that can possibly be made by varying the chemical compounds and their compositions. Later, a detailed experimental characterization of the film is performed by measuring properties such as gas permeability, mechanical strength, anti-microbial activity, and antioxidant properties to name a few. Finally, the testing of the films is carried out at several external environmental conditions. The overall goal is to make an edible coating, strong enough to hold on food items and porous enough to allow transport of oxygen and harmful gases across it. The transport of these gases, one of the critical phenomena determining the effectiveness of the films, is quantified in terms of diffusivity and permeability which are highly dependent on the nano/microstructure of the film. Various factors such as type of compounds (polymers, solvent, etc.), chemical composition, and processing parameters such as pH, drying rate, temperature affect the nano-microstructure of the film.

In order to overcome these limitations, the disclosure herein provides a method and system for developing and characterizing edible films using molecular dynamic simulations. In this approach, a user chosen intent is received via one or more hardware processors, wherein the user chosen intent comprises one of (i) designing of the edible film, and (ii) characterization of the edible film. Further, one or more specifications are received, via the one or more hardware processors, from the user, based on the user chosen intent, wherein the one or more specifications comprise (i) a plurality of design specifications associated with the designing of the edible film, and (ii) a plurality of characterization specifications associated with the characterization of the edible film. Further, a plurality of tunable design factors are assigned via the one or more hardware processors, from the one or more design specifications with associated range of values, based on the user chosen intent. Further, a Design of Experiments (DOE) is generated via the one or more hardware processors, by using a plurality of combinations of the plurality of tunable design factors, wherein the DOE generates a plurality of edible film designs. Further, a molecular model representing a dried version of the edible film is constructed via the one or more hardware processors, for one or more of the plurality of edible film designs. Further, values of one or more properties of the molecular model representing dried film for the one or more of the plurality of edible film designs is estimated using molecular simulations, via the one or more hardware processors, where a list of film properties is provided by user in specifications. Further, if the chosen user intent is designing of film, an optimum value for each of the plurality of tunable design factors, satisfying one or more value requirements of one or more properties of the edible film provided by the user during specifications, is identified via the one or more hardware processors.

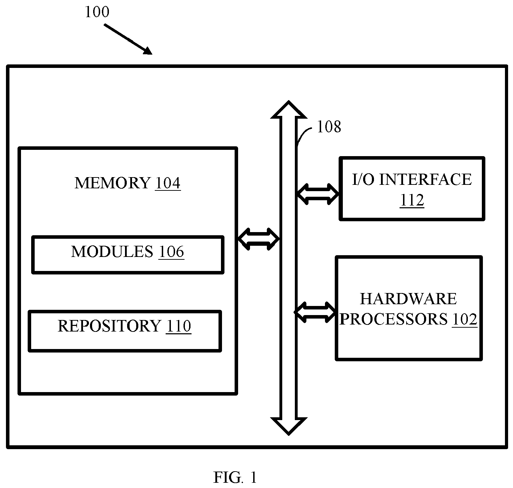

Referring now to the drawings, and more particularly to FIG. 1 through FIG. 7C, where similar reference characters denote corresponding features consistently throughout the figures, there are shown preferred embodiments and these embodiments are described in the context of the following exemplary system and/or method.

FIG. 1 illustrates an exemplary system 100 for design of edible polymer films for coating of perishable food items, according to some embodiments of the present disclosure. The system 100 includes or is otherwise in communication with hardware processors 102, at least one memory such as a memory 104, an I/O interface 112. The hardware processors 102, memory 104, and the Input /Output (I/O) interface 112 may be coupled by a system bus such as a system bus 108 or a similar mechanism. In an embodiment, the hardware processors 102 can be one or more hardware processors.

The I/O interface 112 may include a variety of software and hardware interfaces, for example, a web interface, a graphical user interface, and the like. The I/O interface 112 may include a variety of software and hardware interfaces, for example, interfaces for peripheral device(s), such as a keyboard, a mouse, an external memory, a printer and the like. Further, the I/O interface 112 may enable the system 100 to communicate with other devices, such as web servers, and external databases.

The I/O interface 112 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 112 may include one or more ports for connecting several computing systems with one another or to another server computer. The I/O interface 112 may include one or more ports for connecting several devices to one another or to another server.

The one or more hardware processors 102 may be implemented as one or more microprocessors, microcomputers, microcontrollers, digital signal processors, central processing units, node machines, logic circuitries, and/or any devices that manipulate signals based on operational instructions. Among other capabilities, the one or more hardware processors 102 is configured to fetch and execute computer-readable instructions stored in the memory 104.

The memory 104 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, the memory 104 includes a plurality of modules 106.

The plurality of modules 106 include programs or coded instructions that supplement applications or functions performed by the system 100 for executing different steps involved in the process of switching between hardware accelerators for model training, being performed by the system 100. The plurality of modules 106, amongst other things, can include routines, programs, objects, components, and data structures, which performs particular tasks or implement particular abstract data types. The plurality of modules 106 may also be used as, signal processor(s), node machine(s), logic circuitries, and/or any other device or component that manipulates signals based on operational instructions. Further, the plurality of modules 106 can be used by hardware, by computer-readable instructions executed by the one or more hardware processors 102, or by a combination thereof. The plurality of modules 106 can include various sub-modules (not shown). The plurality of modules 106 may include computer-readable instructions that supplement applications or functions performed by the system 100 for the switching between hardware accelerators for model training.

The data repository (or repository) 110 may include a plurality of abstracted piece of code for refinement and data that is processed, received, or generated as a result of the execution of the plurality of modules in the module(s) 106.

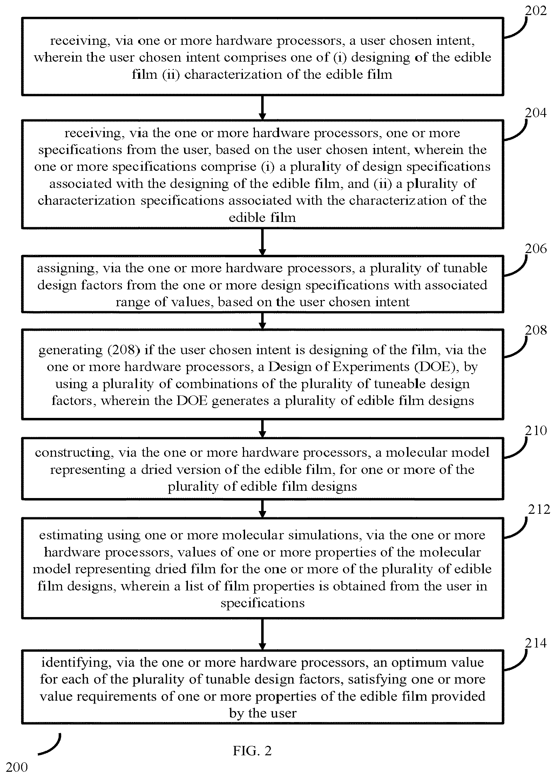

Although the data repository 110 is shown internal to the system 100, it will be noted that, in alternate embodiments, the data repository 110 can also be implemented external to the system 100, where the data repository 110 may be stored within a database (repository 110) communicatively coupled to the system 100. The data contained within such external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS). Functions of the components of the system 100 are now explained with reference to the steps in flow diagrams in FIG. 2, FIG. 3, and FIG. 4.

FIG. 2 is a flow diagram depicting steps involved in the process of design and characterization of edible polymer films for coating of perishable food items, using the system of FIG. 1, according to some embodiments of the present disclosure. In an embodiment, the system 100 comprises one or more data storage devices or the memory 104 operatively coupled to the processor(s) 102 and is configured to store instructions for execution of steps of a method 200 by the processor(s) or one or more hardware processors 102. The steps of the method 200 of the present disclosure will now be explained with reference to the components or blocks of the system 100 as depicted in FIG. 1 and the steps of flow diagram as depicted in FIG. 2. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

At step 202 of method 200 in FIG. 2, a user chosen intent is received via one or more hardware processors, wherein the user chosen intent comprises one of (i) designing of the edible film (ii) characterization of the edible film. The user chosen intent, in other words, is a selection that the user makes, between the designing of the edible film and the characterization of the edible film. Further, at step 204 of the method 200, the system 100 receives one or more specifications, via the one or more hardware processors 102, from the user, based on the user chosen intent, wherein the one or more specifications comprise (i) a plurality of design specifications associated with the designing of the edible film, and (ii) a plurality of characterization specifications associated with the characterization of the edible film. For example, if the user chosen intent is designing of the edible film, the system 100 receives the plurality of design specifications, else if the user chosen intent is the characterization, then the plurality of characterization specifications are received by the system 100.

The plurality of characterization specifications include (i) a plurality of values corresponding to the plurality of tunable design factors, (ii) a plurality of edible film properties to be estimated. The plurality of design specifications include (i) a desired value for at least one of the plurality of edible film properties, (ii) a plurality of values corresponding to the plurality of tunable design factors, (iii) a range of values for at least one of the tunable design factors. The plurality of tunable design factors are factors which affect the plurality of edible film properties, further wherein the plurality of tunable design factors includes, pH, drying rate, drying temperature, polymer properties, acid type and content, plasticizer type and content, dried film water content, and initial water content, wherein the plurality of edible film properties to be estimated comprise transport property, mechanical strength, film morphology. Each of the edible film properties has an associated list of sub-properties.

Further, at step 206 of the method 200, the system 100 assigns a plurality of tunable design factors, via the one or more hardware processors 102, from the one or more design specifications, with associated range of values, based on the user chosen intent. Assigning the plurality of tunable design factors from the one or more specifications with associated range of values includes the following steps.

If the user chosen intent is the designing of the edible film, then the system 100 checks if the range of values corresponding to at least one of the plurality of tunable design factors is provided by the user. If the range of values is obtained as input, a pre-defined number of equally spaced values in the range are assigned to the plurality of tunable design factors, and if the range of values is not obtained as input, the range of values are assigned to the tunable design factors based on pre-existing data. Here, the term pre-existing data may refer to data from a reference database. If the user chosen intent is the characterizing of the edible film, then the system 100 selects middle value of the range of values taken from the pre-existing data for the plurality of tunable design factors whose values are not provided by the user.

Further, at step 208 of the method 200, the system 100 generates a Design of Experiments (DOE) via the one or more hardware processors 102, by using a plurality of combinations of the values of plurality of tunable design factors, wherein the DOE generates a plurality of edible film design. Further, at step 210 of the method 200, a molecular model representing a dried version of the edible film is constructed via the one or more hardware processors 102, for one or more of the plurality of edible film designs.

Further, at step 210 of the method 200, the system 100 constructs the molecular model representing the dried version of the edible film for the one or more of the plurality of edible film designs based on the user chosen intent, by performing the following steps.

Initially, the molecular model representing a dilute solution system is prepared, by obtaining ingredients to prepare an initial molecular system. Obtaining the ingredients to prepare an initial molecular system includes obtaining an initial structure and topology files of the plurality of edible film components and polymer of a predefined chain length based on the design specifications of edible film design from a pre-existing data, followed by obtaining a set of single chain molecular systems of stable protonation pattern by running molecular simulations for a set of molecular box having single chain in vacuum, and then by preparing the molecular system representing dilute solution by running molecular simulations on a box having copies of the set of stable single chains and dimension of the box chosen based on number of water molecules, water density and end-to-end polymer chain distance. Further, a drying process is performed using a molecular simulation of the developed molecular model representing dilute systems, generating a molecular model of the dried film.

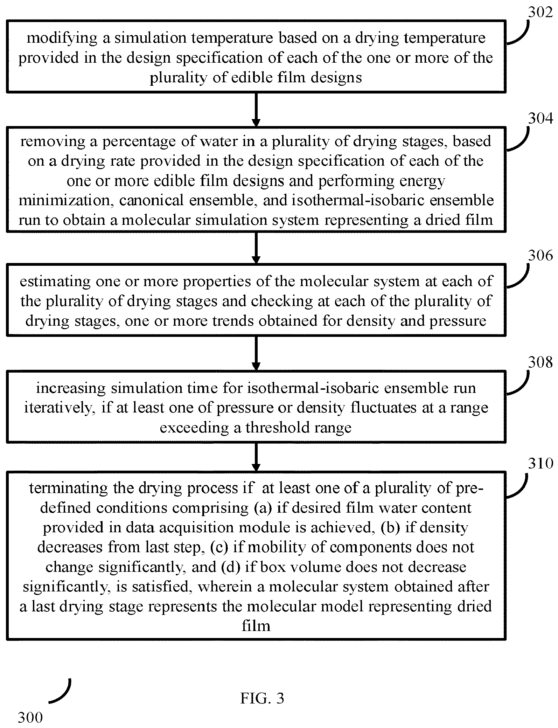

Performing the drying process includes the steps as depicted in method 300 in FIG. 3. In this process, at step 302 of the method 300, a simulation temperature is modified based on a drying temperature provided in the design specification of each of the one or more of the plurality of edible film designs. Further, at step 304 of the method 300, a certain percentage of water is removed in a plurality of drying stages, based on a drying rate provided in the design specification of each of the one or more edible film designs and performing energy minimization, canonical ensemble, and isothermal-isobaric ensemble run to obtain a molecular simulation system representing a dried film. Further, one or more properties of the molecular system are estimated at step 306 of the method 300, at each of the plurality of drying stages and checking at each of the plurality of drying stages, one or more trends obtained for density and pressure. Further, at step 308 of the method 300, a simulation time for isothermal-isobaric ensemble run is increased iteratively, if at least one of pressure or density fluctuates at a range exceeding a threshold range. Further, at step 310 of the method 300, the drying process is terminated if at least one of a plurality of pre-defined conditions comprising (a) if desired film water content provided in data acquisition module is achieved, (b) if density decreases from last step, (c) if mobility of components does not change significantly, and (d) if box volume does not decrease significantly, is satisfied, wherein a molecular system obtained after a last drying stage represents the molecular model representing dried film.

Further, one or more properties of the molecular model representing dried film for the one or more of the plurality of edible film designs is characterized, via the one or more hardware processors 102, using a list of film properties obtained from the user. Referring back to the method 200, further, at step 212 of an optimum value for each of the plurality of tunable design factors, satisfying one or more value requirements of one or more properties of the edible film provided by the user, is identified via the one or more hardware processors 102.

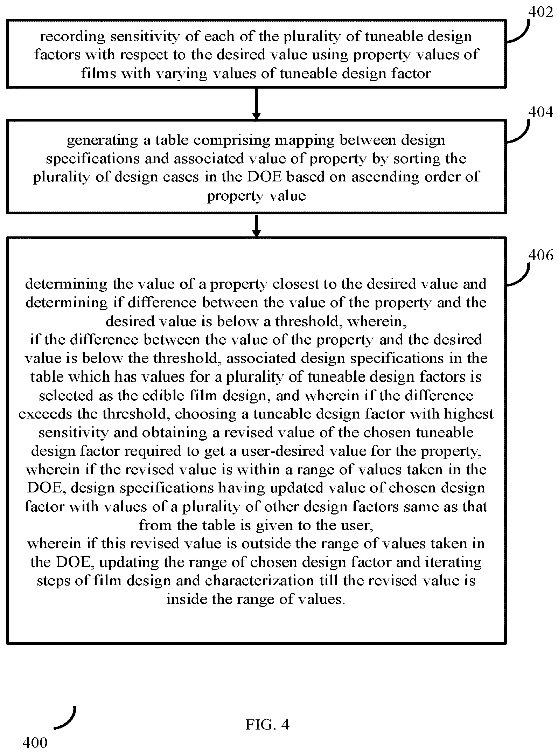

Identifying the optimum value for each of the plurality of tunable design factors includes updating a selected design factor within a selected range, and iteratively performing by increasing the range of a selected design factor, the steps in method 400 in FIG. 4. Initially, at step 402 of the method 400, sensitivity of each of the plurality of tuneable design factors with respect to the property, for which user has specified a desired value, is recorded using property values of films at different values of tunable design factor. Further, at step 404 of the method 400, a table of design specifications versus value of property is obtained by sorting the plurality of design cases in the DOE based on ascending order of property value. Further, at step 406 of the method 400, from the table created above, the value of a property closest to the desired value specified by user is taken, and it is further determined if difference between the value of the property and the desired value is below a threshold, wherein, if the difference is below the threshold, associated design specifications in the table which has values for a plurality of tunable design factors is selected as the edible film design. If the difference exceeds the threshold, then the system 100 obtains new value of chosen tuneable design factor required to get desired value for the property using sensitivity value of that factor (new value_chosen design factor = difference between user-desired and closest value of property/sensitivity). If this revised value is within the range of values taken in DOE, giving design specs to user having updated value of chosen design factor with values of all other design factors same as that taken from table. If this revised value is outside the range, updating the range of chosen design factor and repeating steps of film development and characterization to perform this optimization again, till the revised value is inside the range of values.

Experimental Results:

1) Design specifications provided by user:
a. Edible film components - Water, chitosan
b. Tunable design factors and their values - pH 2.5, 6.5, 9.5

c. Other design specifications - drying temperature 40C

d. Properties of film to be estimated - barrier property (oxygen permeability), film morphology (radius of gyration). An example of the list of sub-properties in each property for the user to choose is shown in Table. 1

e. Desired value of film properties - e.g. tensile strength between 80-120 MPa

Table 1: A list of sub-properties from which the user can choose

| User input | Sub-list of properties |
|---|---|
| Barrier property | Permeability of Oxygen, carbon dioxide, water, ethylene |
| Film morphology | Radius of gyration, end-to-end chain distance, angle distribution |
| Solute-solvent interaction | Solvent-Accessible Surface Area (SASA) SASA, number of contacts, radial distribution function |
| Transport dynamics | Diffusion of compounds in film |
| Mechanical | Tensile strength, elasticity, Young's modulus |

2) For each tunable design factor specified by the user, the system 100 checks if a value or range of values are provided by user: only one tunable design factor specified by the user and it's values are given by user (2.5, 6.5, 9.5) in this example.

3) The system 100 then takes values from pre-built experimental database (Table 2) for drying rate, polymer chain length, etc. to assign the most common value as user has not specified the values for these parameters.

Table 2: Pre-built experimental database comprising range of values and commonly used value of different design factors of film

| Parameter | Relevant range of values | Most commonly used or middle value |
|---|---|---|
| Polymer chain length | 30-120 | 30 |
| Drying rate | 5-15 | 5 |
| Initial water content | 80 - 90% | 85 |
| pH | 2.5 - 10 | 4.5 |
| Film water content | 10 - 40% | 30 |

4) The system 100 then generates a DOE using all possible combinations by taking all values of tunable design factor and the middle value for all other tunable design factors, as shown in Table. 3.

Table 3: DOE

| | Tunable design factor value | Other factor values | | |
|---|---|---|---|---|
| S. No | pH | Drying temperature | Drying rate | Polymer chain length |
| 1 | 2.5 | 40 | 5 | 30 |
| 2 | 6.5 | 40 | 5 | 30 |
| 3 | 9.5 | 40 | 5 | 30 |

5) The system 100 then obtains ingredients and develops a dilute system representing aqueous solution of edible film, executing the following steps:

a. Making a separate folder for the 3 cases in DOE. Repeat steps given below for each folder

b. Copying base files for running MD simulations such as a) structure and forcefield parameter file of solvent, acid and other ingredients from pre-built *component library*, b) mdp files for specifying simulation conditions

c. Adding structure and forcefield file of chitosan for polymer chain length 30 from a pre-built *component library*

d. Choosing a fraction (2/3 in this case) to run single chain simulations in vacuum with each chain assigned random number and arrangement of protonated monomers. The number of monomers are 30 and number of single chain systems are 20. The total number of protonated monomers on all chains out of total monomers (20*30=600) is calculated from Henderson-Hasselbalch Equation (eq. 1) based on pH of system, given in Table 4

$$pH = pK_a + \log_{10} [deprotonated/protonated] \qquad (eq.\ 1)$$

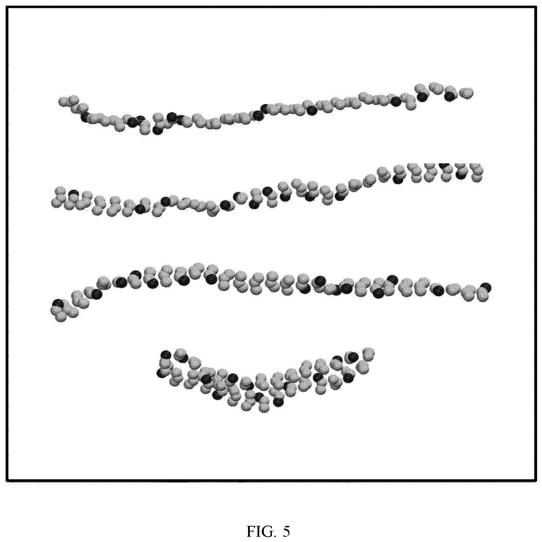

e. Running MD simulations for all single chain systems in vacuum. The representative structures of few of the single chains are shown in Figure 5. It is evident that the chains have different protonation patterns. There were some stable chains which were used for substituting the unstable chains.

f. Taking many solvent beads (~80000 or more) to develop a chitosan-water system. The number of beads is taken such that whole chitosan chain is fully submerged into the water.

g. Calculating number of chains of polymer based on initial water content, chloride beads, water beads taken, molecular weight of polymer, chloride ions and water and copying the set of chains obtained in single-chain simulations by suitable factor.

$$nc*MW\_c = (100\text{-}wc)/100*(nc*MW\_c + nw*4*MW\_w + nCL*MW\_cl)$$

where MW_c, molecular weight of chitosan = 4800 (calculated based on polymer chain length)
MW_w, molecular weight of water = 18
nc, number of chitosan chains
nw, number of water beads = 81600
wc, water content of system = 85%
nCL, number of CL beads = 4800
MW_cl, molecular weight of chloride ions = 72

The number of chains (nc) come out to be 160 for this case, so the 20-set folder was copied 8 times.

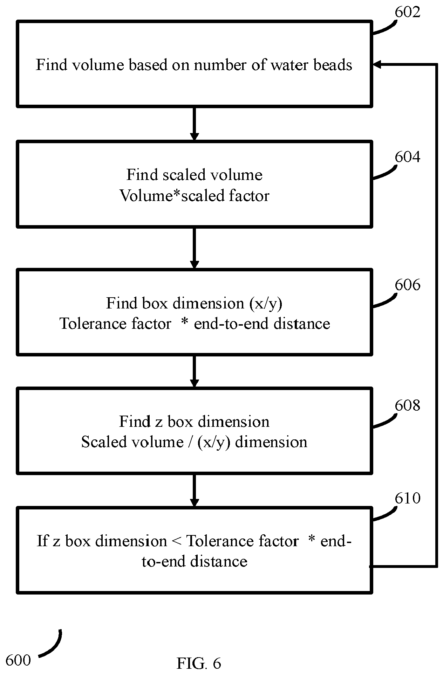

h. Preparing the initial box using dimensions calculated using a pre-defined algorithm (shown in Figure. 6) which estimates the box dimension by considering solvent density and end-to-end polymer chain distance. The box taken is large enough to avoid bad contacts during packing and finite-size effects.

$$Volume\ of\ box = 81600*4*18*10^{27}/(1000*1000*(6.023*10^{23})) = 9754.6\ nm^3$$

End-to-end distance = 12nm (obtained from single chain vacuum simulation)
Scale factor = 1.3
Tolerance factor = 1.3

$$Scaled\ volume = 1.3 * 9754.6 = 12680.9\ nm^3$$

$$x/y\ dimension = 12*1.3 = 16nm$$

$$z\text{-}dimension = 12680.9/(16*16) = 49nm$$

i. Using the initial box configuration obtained to do energy minimization, canonical ensemble and isothermal-isobaric ensemble run

j. If simulations are unstable, water beads in the system are too few leading to voids in system. In this case, simulations run successfully.

6. The system 100 then performs the drying process on dilute system obtained in last step to develop dried film, by executing the following steps:

i. Setting up the simulation parameters such as temperature (40C)

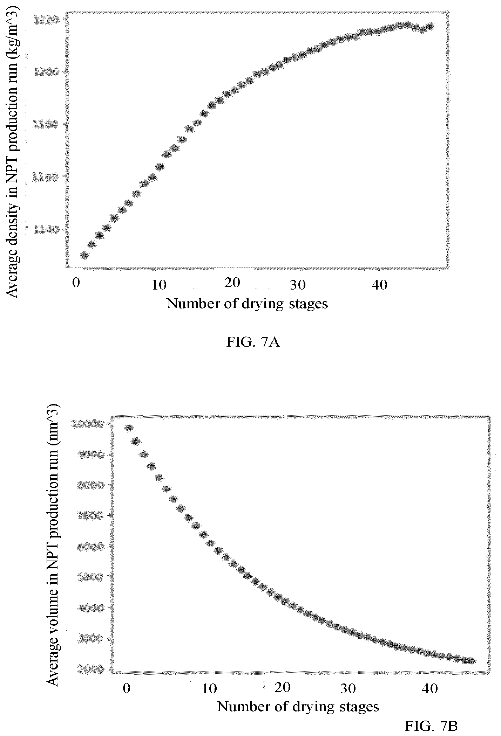

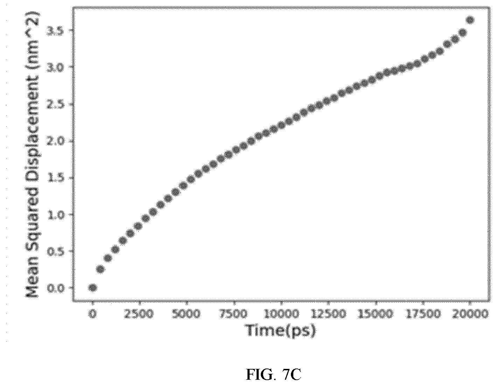

ii. Using a python script to perform drying by removing 5% percentage of water at every stage and further performing energy minimization, and then MD simulation at constant number of molecules, volume of the box and temperature (also known as canonical ensemble) and later at constant number of molecules, pressure, and temperature (also known as isothermal-isobaric ensemble), to estimate basic properties such as density and pressure. Modifying the values of drying rate (equal to 5%) and desired film water content (equal to 30%) in the script

iii. The convergence of density is checked as the equilibration of the system. The density is monitored throughout the simulation and once the density is equilibrated, the system is simulated further for 10 ns of nanoseconds of production runs

iv. Stop drying process if some pre-defined conditions are met for example a) if desired film water content is achieved, b) if density decreases from last step, c) if box volume does not decrease significantly. As density and volume were continuously decreasing for all the cases (shown for pH 6.5 in FIGS. 7A, 7B, and 7C), drying was stopped when desired water content was achieved

7. The system 100 then characterizes the molecular systems obtained for dried films, executing the following steps:

a. list of film properties (barrier property (oxygen permeability), film morphology) are obtained from user specified specifications

b. taking simulation files of dried film obtained in last step

c. running python script where functions for estimation of different properties that can be obtained from MD simulation are written. This python script was run based on list of properties required by the user and the results were provided to the user. Here, the estimation of oxygen permeability was done by multiplying solubility and diffusivity. For diffusivity calculation, the structure file of dried film of each case in DOE was taken and 10 molecules in case of oxygen were randomly inserted in the film box to create 20 different systems for obtaining averaged Mean Squared Displacement (MSD) of oxygen. Each system was equilibrated and canonical ensemble of 20ns and isobaric-isothermal ensemble of 20ns is run. The timestep used for the simulations was 20fs. The insertion of oxygen gas molecules and initial velocity generation for given temperature were done using different seed numbers for different systems. The average of the MSD obtained using all 20 systems is used for diffusivity calculation. The graph obtained for MSD is shown in Figure 4. For solubility calculation, the structure file of dried film for each case in DOE was taken and 1 molecule of oxygen gas was inserted in the film box. The equilibrated film box was taken for free energy calculation. 20 equally spaced steps were taken for tuning Vanderwal forces in free-energy calculation. Since oxygen course-grained model does not have a charge, only Vanderwal forces are tuned. The derivatives of Hamiltonian obtained at all steps are summed to obtain the free energy of solvation. The value of solubility from free energy of solvation is estimated using equation:

$$\text{Solubility (S)} = \beta \exp (- \beta \ \Delta G)$$

Where $\beta$ is $1/(R*T)$, R is ideal gas constant, T is temperature and $\Delta G$ is free energy of solvation. The dried chitosan film obtained for the three cases in DOE was used for characterization. The estimated properties based on user inputs are shown in Table 5. These properties were also validated with experimental studies in literature and comparison is shown in Table 6.

Table 4: Distribution of protonated monomers based on pH of the system

| pH | Total no of monomers | Number of deprotonated | Number of protonated |
|---|---|---|---|
| 2.9 | 600 | 600 | 0 |
| 6.5 | 600 | 300 | 300 |
| 9.3 | 600 | 10 | 590 |

Table 5: The table shows results for properties estimated for different systems

| Tunable input factor (pH) | Oxygen permeability (g/m s Pa) | Radius of gyration (nm) |
|---|---|---|
| 2.9 | $1.01 \times 10^{-14}$ | 10.1 |
| 6.5 | $2.86 \times 10^{-14}$ | 9 |
| 9.3 | $1.91 \times 10^{-13}$ | 8.3 |

Table 6: The validation of properties obtained in MD simulations with those available in literature experimental studies

| Property | Current study | Literature |
|---|---|---|
| Radius of gyration | 8.66-8.76 nm | 7.93 nm (based on next slide formula) |
| Density | 1210 kg/m$^3$ for water content 35% | 1400 kg/m$^3$ for water content 12-20% [1] |
| Oxygen permeability | 1 E-14 to 3 E-14 g/m s Pa | 1.1 E-14 g/m Pa s [7] |

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address unresolved problem of edible food coating design. The embodiment, thus provides a mechanism for edible food coating design.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method (200), comprising:

   receiving (202), via one or more hardware processors, a user chosen intent, wherein the user chosen intent comprises one of (i) designing of the edible film (ii) characterization of the edible film;

   receiving (204), via the one or more hardware processors, one or more specifications from the user, based on the user chosen intent, wherein the one or more specifications comprise (i) a plurality of design specifications associated with the designing of the edible film, and (ii) a plurality of characterization specifications associated with the characterization of the edible film;

   assigning (206), via the one or more hardware processors, a plurality of tunable design factors from the one or more design specifications with associated range of values, based on the user chosen intent;

   generating (208), via the one or more hardware processors, a Design of Experiments (DOE), by using a plurality of combinations of the plurality of tunable design factors, wherein the DOE generates a plurality of edible film designs, if the user chosen intent is designing of the film;

   constructing (210), via the one or more hardware processors, a molecular model representing a dried version of the edible film, for one or more of the plurality of edible film designs;

   estimating using one or more molecular simulations (212), via the one or more hardware processors, values of one or more properties of the molecular model representing dried film for the one or more of the plurality of edible film designs, wherein a list of film properties is obtained from the user in the one or more specifications; and

   identifying (214), via the one or more hardware processors, an optimum value for each of the plurality of tunable design factors, satisfying one or more value requirements of one or more properties of the edible film provided by the user, if the user chosen intent is designing of the film.

2. The processor implemented method as claimed in claim 1, wherein,
   the plurality of characterization specifications comprises of (i) a plurality of values corresponding to the plurality of tunable design factors, and (ii) a plurality of edible film properties to be estimated,

   wherein, the plurality of design specifications comprises of (i) a desired value for at least one of the plurality of edible film properties, (ii) a plurality of values associated with the plurality of tunable design factors, and (iii) a range of values for at least one of the plurality of tunable design factors,

   wherein, the plurality of tunable design factors are factors which affect the plurality of edible film properties, further wherein the plurality of tunable design factors comprises of pH, drying rate, drying temperature, polymer properties, acid type and content, plasticizer type and content, dried film water content, and initial water content, and

   wherein, the plurality of edible film properties to be estimated comprise transport property, mechanical strength, film morphology, wherein each of the edible film properties has an associated list of sub-properties.

3. The processor implemented method as claimed in claim 1, wherein assigning the plurality of tunable design factors from the one or more specifications with associated range of values comprises:
   if the user chosen intent is the designing of the edible film:

   checking, if the range of values corresponding to at least one of the plurality of tunable design factors is provided by the user,

   wherein if the range of values is obtained as input, a pre-defined number of equally spaced values in the range are assigned to the plurality of tunable design factors, and

   wherein if the range of values is not obtained as input, the range of values are assigned to the tunable design factors based on pre-existing data, and

   if the user chosen intent is the characterization of the edible film:

   selecting middle value of the range of values taken from the pre-existing data for the plurality of tunable design factors for which values are not provided by the user.

4. The processor implemented method as claimed in claim 1, wherein constructing the molecular model representing the dried version of the edible film for the one or more of the plurality of edible film designs based on the user chosen intent comprises:
   preparing the molecular model representing a dilute solution system, comprising:

   obtaining ingredients to prepare an initial molecular system, by:

obtaining, an initial structure and topology files of the plurality of edible film components and polymer of a predefined chain length based on the design specifications of edible film design from a pre-existing data; obtaining a set of single chain molecular systems of stable protonation pattern by running molecular simulations for a set of molecular box having single chain in vacuum; and

preparing the molecular system representing dilute solution by running molecular simulations on a box having copies of the set of stable single chains and dimension of the box chosen based on number of water molecules, water density and end-to-end polymer chain distance; and performing a drying process using a molecular simulation of the developed molecular model representing dilute systems, generating a molecular model of the dried film.

5. The processor implemented method as claimed in claim 4, wherein performing the drying process comprises:

modifying (302) a simulation temperature based on a drying temperature provided in the design specification of each of the one or more of the plurality of edible film designs;
removing (304) a percentage of water in a plurality of drying stages, based on a drying rate provided in the design specification of each of the one or more edible film designs and performing energy minimization, canonical ensemble, and isothermal-isobaric ensemble run to obtain a molecular simulation system representing a dried film;
estimating (306) one or more properties of the molecular system at each of the plurality of drying stages and checking at each of the plurality of drying stages, one or more trends obtained for density and pressure;
increasing (308) simulation time for isothermal-isobaric ensemble run iteratively, if at least one of pressure or density fluctuates at a range exceeding a threshold range; and
terminating (310) the drying process if at least one of a plurality of pre-defined conditions comprising (a) if desired film water content provided in data acquisition module is achieved, (b) if density decreases from last step, (c) if mobility of components does not change significantly, and (d) if box volume does not decrease significantly, is satisfied, wherein a molecular system obtained after a last drying stage represents the molecular model representing dried film.

6. The processor implemented method as claimed in claim 1, wherein identifying the optimum value for each of the plurality of tunable design factors comprises updating a selected design factor within a selected range and iteratively performing by increasing the range of a selected design factor, comprising:

recording (402) sensitivity of each of the plurality of tuneable design factors with respect to the desired value using property values of films with varying values of tuneable design factor;
generating (404) a table comprising mapping between design specifications and associated value of property by sorting the plurality of design cases in the DOE based on ascending order of property value; and
determining (406) the value of a property closest to the desired value and determining if difference between the value of the property and the desired value is below a threshold, wherein,

if the difference between the value of the property and the desired value is below the threshold, associated design specifications in the table which has values for a plurality of tuneable design factors is selected as the edible film design, and
wherein if the difference exceeds the threshold,

choosing a tuneable design factor with highest sensitivity and obtaining a revised value of the chosen tuneable design factor required to get a user-desired value for the property,
wherein if the revised value is within a range of values taken in the DOE, design specifications having updated value of chosen design factor with values of a plurality of other design factors same as that from the table is given to the user, and
wherein if this revised value is outside the range of values taken in the DOE, updating the range of chosen design factor and iterating steps of film design and characterization till the revised value is inside the range of values.

7. A system (100), comprising:

one or more hardware processors (102);
a communication interface (112); and

a memory (104) storing a plurality of instructions, wherein the plurality of instructions when executed, cause the one or more hardware processors to:

receive a user chosen intent, wherein the user chosen intent comprises one of (i) designing of the edible film (ii) characterization of the edible film;

receive one or more specifications from the user, based on the user chosen intent, wherein the one or more specifications comprise (i) a plurality of design specifications associated with the designing of the edible film, and (ii) a plurality of characterization specifications associated with the characterization of the edible film;

assign a plurality of tunable design factors from the one or more design specifications with associated range of values, based on the user chosen intent;

generate a Design of Experiments (DOE), by using a plurality of combinations of the plurality of tunable design factors, wherein the DOE generates a plurality of edible film designs, if the user chosen intent is designing of the film;

construct a molecular model representing a dried version of the edible film, for one or more of the plurality of edible film designs;

estimate using one or more molecular simulations, values of one or more properties of the molecular model representing dried film for the one or more of the plurality of edible film designs, wherein a list of film properties is obtained from the user in specifications; and

identify an optimum value for each of the plurality of tunable design factors, satisfying one or more value requirements of one or more properties of the edible film provided by the user, if the user chosen intent is designing of the film.

8. The system as claimed in claim 7, wherein,
the plurality of characterization specifications comprises of (i) a plurality of values corresponding to the plurality of tunable design factors, and (ii) a plurality of edible film properties to be estimated,

wherein, the plurality of design specifications comprises of (i) a desired value for at least one of the plurality of edible film properties, (ii) a plurality of values associated with the plurality of tunable design factors, and (iii) a range of values for at least one of the plurality of tunable design factors,

wherein, the plurality of tunable design factors are factors which affect the plurality of edible film properties, further wherein the plurality of tunable design factors comprises of pH, drying rate, drying temperature, polymer properties, acid type and content, plasticizer type and content, dried film water content, and initial water content, and

wherein, the plurality of edible film properties to be estimated comprise transport property, mechanical strength, film morphology, wherein each of the edible film properties has an associated list of sub-properties.

9. The system as claimed in claim 7, wherein the one or more hardware processors are configured to assign the plurality of tunable design factors from the one or more specifications with associated range of values, by:

if the user chosen intent is the designing of the edible film:

checking, if the range of values corresponding to at least one of the plurality of tunable design factors is provided by the user,

wherein if the range of values is obtained as input, a pre-defined number of equally spaced values in the range are assigned to the plurality of tunable design factors, and

wherein if the range of values is not obtained as input, the range of values are assigned to the tunable design factors based on pre-existing data, and

if the user chosen intent is the characterization of the edible film:
selecting middle value of the range of values taken from the pre-existing data for the plurality of tunable design factors for which values are not provided by the user.

10. The system as claimed in claim 7, wherein the one or more hardware processors are configured to construct the molecular model representing the dried version of the edible film for the one or more of the plurality of edible film designs based on the user chosen intent, by:

preparing the molecular model representing a dilute solution system, comprising:
obtaining ingredients to prepare an initial molecular system, by:

obtaining, an initial structure and topology files of the plurality of edible film components and polymer of a predefined chain length based on the design specifications of edible film design from a pre-existing data; obtaining a set of single chain molecular systems of stable protonation pattern by running molecular simulations for a set of molecular box having single chain in vacuum; and preparing the molecular system representing dilute solution by running molecular simulations on a box having copies of the set of stable single chains and dimension of the box chosen based on number of water molecules, water density and end-to-end polymer chain distance; and

performing a drying process using a molecular simulation of the developed molecular model representing dilute systems, generating a molecular model of the dried film.

11. The system as claimed in claim 10, wherein the one or more hardware processors are configured to perform the drying process by:

modifying a simulation temperature based on a drying temperature provided in the design specification of each of the one or more of the plurality of edible film designs;
removing a percentage of water in a plurality of drying stages, based on a drying rate provided in the design specification of each of the one or more edible film designs and performing energy minimization, canonical ensemble, and isothermal-isobaric ensemble run to obtain a molecular simulation system representing a dried film;
estimating one or more properties of the molecular system at each of the plurality of drying stages and checking at each of the plurality of drying stages, one or more trends obtained for density and pressure;
increasing simulation time for isothermal-isobaric ensemble run iteratively, if at least one of pressure or density fluctuates at a range exceeding a threshold range; and
terminating the drying process if at least one of a plurality of pre-defined conditions comprising (a) if desired film water content provided in data acquisition module is achieved, (b) if density decreases from last step, (c) if mobility of components does not change significantly, and (d) if box volume does not decrease significantly, is satisfied, wherein a molecular system obtained after a last drying stage represents the molecular model representing dried film.

12. The system as claimed in claim 7, wherein the one or more hardware processors are configured to identify the optimum value for each of the plurality of tunable design factors by updating a selected design factor within a selected range and iteratively performing by increasing the range of a selected design factor, by:

recording sensitivity of each of the plurality of tuneable design factors with respect to the desired value using property values of films with varying values of tuneable design factor;
generating a table comprising mapping between design specifications and associated value of property by sorting the plurality of design cases in the DOE based on ascending order of property value; and
determining the value of a property closest to the desired value and determining if difference between the value of the property and the desired value is below a threshold, wherein,

if the difference between the value of the property and the desired value is below the threshold, associated design specifications in the table which has values for a plurality of tuneable design factors is selected as the edible film design, and
wherein if the difference exceeds the threshold,

choosing a tuneable design factor with highest sensitivity and obtaining a revised value of the chosen tuneable design factor required to get a user-desired value for the property,
wherein if the revised value is within a range of values taken in the DOE, design specifications having updated value of chosen design factor with values of a plurality of other design factors same as that from the table is given to the user, and
wherein if this revised value is outside the range of values taken in the DOE, updating the range of chosen design factor and iterating steps of film design and characterization till the revised value is inside the range of values.

13. One or more non-transitory machine-readable information storage mediums comprising one or more instructions which when executed by one or more hardware processors cause:

receiving a user chosen intent, wherein the user chosen intent comprises one of (i) designing of the edible film (ii) characterization of the edible film;

receiving one or more specifications from the user, based on the user chosen intent, wherein the one or more specifications comprise (i) a plurality of design specifications associated with the designing of the edible film, and (ii) a plurality of characterization specifications associated with the characterization of the edible film;

assigning a plurality of tunable design factors from the one or more design specifications with associated range of values, based on the user chosen intent; generating a Design of Experiments (DOE), by using a plurality of combinations of the plurality of tunable design factors, wherein the DOE generates a plurality of edible film designs, if the user chosen intent is designing of the film;

constructing a molecular model representing a dried version of the edible film, for one or more of the plurality of edible film designs;

estimating using one or more molecular simulations, values of one or more properties of the molecular model representing dried film for the one or more of the plurality of edible film designs, wherein a list of film properties is obtained from the user in the one or more specifications; and

identifying an optimum value for each of the plurality of tunable design factors, satisfying one or more value requirements of one or more properties of the edible film provided by the user, if the user chosen intent is designing of the film.

14. The one or more non-transitory machine-readable information storage mediums of claim 13, wherein,

the plurality of characterization specifications comprises of (i) a plurality of values corresponding to the plurality of tunable design factors, and (ii) a plurality of edible film properties to be estimated,

wherein, the plurality of design specifications comprises of (i) a desired value for at least one of the plurality of edible film properties, (ii) a plurality of values associated with the plurality of tunable design factors, and (iii) a range of values for at least one of the plurality of tunable design factors,

wherein, the plurality of tunable design factors are factors which affect the plurality of edible film properties, further wherein the plurality of tunable design factors comprises of pH, drying rate, drying temperature, polymer properties, acid type and content, plasticizer type and content, dried film water content, and initial water content, and

wherein, the plurality of edible film properties to be estimated comprise transport property, mechanical strength, film morphology, wherein each of the edible film properties has an associated list of sub-properties.

15. The one or more non-transitory machine-readable information storage mediums of claim 13, wherein assigning the plurality of tunable design factors from the one or more specifications with associated range of values comprises:

if the user chosen intent is the designing of the edible film:

checking, if the range of values corresponding to at least one of the plurality of tunable design factors is provided by the user,

wherein if the range of values is obtained as input, a pre-defined number of equally spaced values in the range are assigned to the plurality of tunable design factors, and

wherein if the range of values is not obtained as input, the range of values are assigned to the tunable design factors based on pre-existing data, and

if the user chosen intent is the characterization of the edible film:

selecting middle value of the range of values taken from the pre-existing data for the plurality of tunable design factors for which values are not provided by the user.

FIG. 1

receiving, via one or more hardware processors, a user chosen intent, wherein the user chosen intent comprises one of (i) designing of the edible film (ii) characterization of the edible film                      202

receiving, via the one or more hardware processors, one or more specifications from the user, based on the user chosen intent, wherein the one or more specifications comprise (i) a plurality of design specifications associated with the designing of the edible film, and (ii) a plurality of characterization specifications associated with the characterization of the edible film                      204

assigning, via the one or more hardware processors, a plurality of tunable design factors from the one or more design specifications with associated range of values, based on the user chosen intent                      206

generating (208) if the user chosen intent is designing of the film, via the one or more hardware processors, a Design of Experiments (DOE), by using a plurality of combinations of the plurality of tuneable design factors, wherein the DOE generates a plurality of edible film designs                      208

constructing, via the one or more hardware processors, a molecular model representing a dried version of the edible film, for one or more of the plurality of edible film designs                      210

estimating using one or more molecular simulations, via the one or more hardware processors, values of one or more properties of the molecular model representing dried film for the one or more of the plurality of edible film designs, wherein a list of film properties is obtained from the user in specifications                      212

identifying, via the one or more hardware processors, an optimum value for each of the plurality of tunable design factors, satisfying one or more value requirements of one or more properties of the edible film provided by the user                      214

FIG. 2

200

302

modifying a simulation temperature based on a drying temperature provided in the design specification of each of the one or more of the plurality of edible film designs

304

removing a percentage of water in a plurality of drying stages, based on a drying rate provided in the design specification of each of the one or more edible film designs and performing energy minimization, canonical ensemble, and isothermal-isobaric ensemble run to obtain a molecular simulation system representing a dried film

306

estimating one or more properties of the molecular system at each of the plurality of drying stages and checking at each of the plurality of drying stages, one or more trends obtained for density and pressure

308

increasing simulation time for isothermal-isobaric ensemble run iteratively, if at least one of pressure or density fluctuates at a range exceeding a threshold range

310

terminating the drying process if at least one of a plurality of pre-defined conditions comprising (a) if desired film water content provided in data acquisition module is achieved, (b) if density decreases from last step, (c) if mobility of components does not change significantly, and (d) if box volume does not decrease significantly, is satisfied, wherein a molecular system obtained after a last drying stage represents the molecular model representing dried film

FIG. 3

300

402

recording sensitivity of each of the plurality of tuneable design factors with respect to the desired value using property values of films with varying values of tuneable design factor

404

generating a table comprising mapping between design specifications and associated value of property by sorting the plurality of design cases in the DOE based on ascending order of property value

406

determining the value of a property closest to the desired value and determining if difference between the value of the property and the desired value is below a threshold, wherein,
if the difference between the value of the property and the desired value is below the threshold, associated design specifications in the table which has values for a plurality of tuneable design factors is selected as the edible film design, and wherein if the difference exceeds the threshold, choosing a tuneable design factor with highest sensitivity and obtaining a revised value of the chosen tuneable design factor required to get a user-desired value for the property, wherein if the revised value is within a range of values taken in the DOE, design specifications having updated value of chosen design factor with values of a plurality of other design factors same as that from the table is given to the user,
wherein if this revised value is outside the range of values taken in the DOE, updating the range of chosen design factor and iterating steps of film design and characterization till the revised value is inside the range of values.

400

FIG. 4

FIG. 5

602

Find volume based on number of water beads

604

Find scaled volume
Volume*scaled factor

606

Find box dimension (x/y)
Tolerance factor  * end-to-end distance

608

Find z box dimension
Scaled volume / (x/y) dimension

610

If z box dimension < Tolerance factor  * end-to-end distance

600

FIG. 6

FIG. 7A

FIG. 7B

FIG. 7C

## EUROPEAN SEARCH REPORT

Application Number

EP 23 20 4948

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SISTLA YAMINI SUDHA ET AL: "Molecular Simulations to Understand the Moisture, Carbon Dioxide, and Oxygen Barrier Properties of Pectin Films", JOURNAL OF MOLECULAR MODELING, SPRINGER BERLIN HEIDELBERG, BERLIN/HEIDELBERG, vol. 28, no. 4, 5 March 2022 (2022-03-05), XP037709329, ISSN: 1610-2940, DOI: 10.1007/S00894-022-05069-Z [retrieved on 2022-03-05] * abstract * * page 2, column 1, paragraph 2 * ----- | 1-15 | INV. G16C60/00 |
| X | VUDDANDA PARAMESWARA RAO ET AL: "Effect of plasticizers on the physico-mechanical properties of pullulan based pharmaceutical oral films", EUROPEAN JOURNAL OF PHARMACEUTICAL SCIENCES, ELSEVIER AMSTERDAM, NL, vol. 96, 11 September 2016 (2016-09-11), pages 290-298, XP029833815, ISSN: 0928-0987, DOI: 10.1016/J.EJPS.2016.09.011 * abstract * * Conclusions * ----- | 1-15 | |
| | | | **TECHNICAL FIELDS SEARCHED (IPC)** |
| | | | G16C |
| | -/-- | | |

The present search report has been drawn up for all claims

| Place of search | Date of completion of the search | Examiner |
|---|---|---|
| The Hague | 15 April 2024 | Nurmi, Jussi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 23 20 4948

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ÖZEREN HÜSAMETTIN D ET AL: "Prediction of plasticization in a real biopolymer system (starch) using molecular dynamics simulations", MATERIALS & DESIGN, ELSEVIER, AMSTERDAM, NL, vol. 187, 27 November 2019 (2019-11-27), XP085977243, ISSN: 0264-1275, DOI: 10.1016/J.MATDES.2019.108387 [retrieved on 2019-11-27] * abstract * * Conclusions * | 1-15 | |

-----

TECHNICAL FIELDS
SEARCHED      (IPC)

The present search report has been drawn up for all claims

| Place of search | Date of completion of the search | Examiner |
|---|---|---|
| The Hague | 15 April 2024 | Nurmi, Jussi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................
& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202221076873